# EUROPEAN PATENT SPECIFICATION

(11) **EP 2 758 423 B1**
(45) Date of publication and mention of the grant of the patent: **19.08.2020**
(21) Application number: 12791277.2
(22) Date of filing: 18.09.2012
(51) Int. Cl.: C07K 14/415, A61K 38/16, A61P 1/00

(54) **PEPTIDES HAVING PROTECTIVE EFFECT TOWARDS THE INFLAMMATORY ACTIVITY OF PEPTIDE 31-43 OF A-GLIADIN IN CELIAC DISEASE**
PEPTIDE MIT EINER SCHUTZWIRKUNG GEGEN DIE ENTZÜNDLICHE AKTIVITÄT DES PEPTIDES 31-43 DES A-GLIADINS IN ZÖLIAKIE
PEPTIDES AYANT UN EFFET PROTECTEUR VIS-À-VIS DE L'ACTIVITÉ INFLAMMATOIRE D'UN PEPTIDE 31-43 D'A-GLIADINE DANS UNE MALADIE COELIAQUE

(30) Priority: 19.09.2011 IT RM20110487
(43) Date of publication of application: 30.07.2014
(73) Proprietor: Istituto Superiore di Sanità, 00161 Roma (IT); CRA-Consiglio per la Ricerca e la Sperimentazione in Agricoltura, 00184 Roma (IT)
(72) Inventor: SILANO, Marco, 00135 Roma (RM) (IT); CATTIVELLI, Luigi, 00184 Roma (IT); DE VITA, Pasquale, 00184 Roma (IT); MAIURI, Luigi, 00184 Roma (IT); FICCO, Donatella Bianca Maria, 00184 Roma (IT)
(74) Representative: Gitto, Serena
(86) International application number: PCT/IT2012/000287
(87) International publication number: WO 2013/042156

(56) References cited:
- WO-A1-2010/060155
- CHAI JIAN FANG ET AL: "Homoeologous cloning of omega-secalin gene family in a wheat 1BL/1RS translocation.", CELL RESEARCH AUG 2005 LNKD- PUBMED:16117855, vol. 15, no. 8, August 2005 (2005-08), pages 658-664, XP002677707, ISSN: 1001-0602
- Silano: "A 10-residue peptide from durum wheat promotes a shift from a Th1-type response toward a Th2-type response in celiac disease", , 1 February 2008 (2008-02-01), XP055407596, Retrieved from the Internet: URL:http://ajcn.nutrition.org/content/87/2 /415.full.pdf [retrieved on 2017-09-18]

## Description

The present invention concerns peptides having protective effect towards inflammatory activity of a-gliadin 31-43 peptide in celiac disease. Particularly, the invention concerns peptides having protective effect towards inflammatory activity of a-gliadin 31-43 peptide in celiac disease and therefore said peptides can be used for preventive and therapeutic purpose by administration thereof to subjects at high risk to develop celiac disease and/or celiac subjects just before a gluten containing meal to be ingested.

Celiac disease (CD) is a permanent autoimmune enteropathy, triggered by gluten ingestion in genetically prone subjects. Gluten is alcohol-soluble portion of wheat, rye and barley. The main protein of wheat gluten is gliadin, respective prolamins of barley and rye are ordein and secalin (1-3).

During the last few years, various experimental evidences relating to role of native immunity in priming and supporting bowel mucosal inflammation in celiac subjects have been reported. Particularly, in order to allow the *in vitro* activation of gliadin-specific T CD4+ lymphocytes by immunodominant gliadin peptides, like α-9 and α-2, pre-incubation of duodenal celiac mucosa with peptide containing amino acids at positions 31-43 within gliadin sequence (p31-43) is necessary. Moreover, only toxic peptide 31-43, but not immunodominant ones, displayed activity *in vitro* to induce fast expression of IL-15, CD83, COX-2, apoptosis of enterocytes and activation of p38 MAP kinase in celiac mucosa (4). Recently, it has been reported that only toxic 31-43 peptide, but not immunodominant ones, is retained within the endosomal lysosomal system of enterocytes and results in intracellular increase of oxygen reactive species (ROS) inhibiting TG2 ubiquinitation, that in turn, results in an increase of TG2 levels and activity. Then TG2 determines cross-linking, ubiquinitation and degradation of PPAPγ (5).

Unique currently available treatment for CD is gluten free diet over all the life. This is a therapy remarkably limiting the life quality of celiac subjects, however a strict patient compliance in order to prevent harmful consequences of gluten consumption in celiac subjects is necessary.

In the light of above it is therefore apparent the need to provide for new therapeutic strategies as an alternative to gluten free diet.

In the past within wheat peptic-triptic digest a decapeptide with QQPQDAVQPF (SEQ ID NO: 1) sequence (WO9727217), below named pDAV, has been identified. This peptide has been displayed suitable to prevent the agglutination of K562(S) cells by gliadin peptides; to prevent the activation of epithelial intestinal cells and inhibit cellular Th1 celiac response (6-9). Initially it was thought that pDAV peptide could be used for CD treatment by administration of pDAV along with gluten in order the latter to be tolerated by celiac subjects. However, successive studies up to now were unsuccessful to identify in wheat genome the gene encoding for protein containing pDAV sequence. It is possible that said sequence has been originated by a gene mutation carried by the specific plant from which the flour sample analyzed at laboratories of Istituto Superiore di Sanità in 1997 wherein pDAV peptide was identified, had been extracted. Therefore, the hypothesis to use pDAV for therapeutic purpose was abandoned in the absence of a sure evidence of the naturally occurrence of this peptide. The use of pDAV in therapy in fact would have exposed the patients to possible risks deriving from treatment with a peptide to which population normally is not exposed.

The authors of the present invention have identified a nucleotide sequence encoding for a peptide similar to pDAV in terms of sequence and functionally equivalent to pDAV. Said new peptide has QQPQRPQQPF (SEQ ID NO: 2) sequence, from now on named pRPQ. pRPQ corresponds to positions 218-227 of ω-secalin codified by the sequence deposited at NCBI database with FJ823444 accession number. Same peptide was found also in the sequences of ω-secalin FJ823438 (amino acids at positions 186-195), FJ823439 (positions 101-110), FJ823443 (positions 85-94), FJ823441 (positions 86-95), FJ823440 (positions 85-94) and FJ823442 (positions 157-166). All the described sequences have been isolated by the authors of the present invention using Kavkaz tender wheat peptide. Same peptide moreover has been found within three previously database deposited sequences (X60294 and X60295 encoding for ω-secalin and isolated from *Secale cereale* and AF000227 encoding for ω-secalin and isolated from *Triticum aestivum*).

pDAV and pRPQ peptides share seven amino acids (QQPQQPF, SEQ ID NO: 3) (7mer) and both contain the insertion of amino acid triplet between QQPQ and QPF domains. It is to be pointed out that 7mer peptide is known to be toxic in CD (10). The authors of the present invention have investigated whether pRPQ had the same protecting activity like pDAV towards gluten in CD and whether QQPF and QPF spacing in 7mer sequence was suitable to result in a protecting activity of resulting peptide. At this end the model of K562(S) cell agglutination has been used the latter being a quick and validated system to study gliadin peptide toxicity in CD (11).

Therefore it has been verified that pRPQ peptide, whose sequence occurs naturally in tender wheat varieties characterized by the introgression of part of rye chromosome 1R short arm, not only is not toxic for celiac subjects, but in addition displays also a protecting effect against toxicity of p31-43 peptide. This effect has been estimated on three different *in vitro* CD systems, all validated according to reported literature data, including the culture of intestinal mucosa cells, that is considered golden standard for evaluation of possible therapies for CD. It has been found moreover that peptide protecting effect results from spacing between said two QQPF and QPF domains. Moreover larger the spacing, higher the peptide protecting effect. The peptide obtained with insertion of a W, i.e. a large size amino acid, within p7mer results in the same protecting activity as obtained with the insertion of three G, i.e. a small size amino acid. An intermediate protecting effect resulted from the insertion of GG or YYY. Therefore, while the patent application WO9727217 postulated that pDAV protecting activity was due to specific amino acid sequence and in addition suggested that a minimum sequence of 5 amino acids was necessary in order to obtain said protecting effect, the authors of the present invention found that effectively the peptide protecting effect results from the spacing between QQPF and QPF domains and a greater spacing corresponds to higher protecting effect by the peptide. The peptides of the invention are suitable to prevent agglutination of K562(S) cells, expression of ICAM-1 in T84 cells, phosphorylation of p42/44 and activity of TG2 in the duodenal mucosa. Therefore, the peptides of the invention can be advantageously used for preventive and therapeutic purpose by administration thereof to subjects at high risk to develop CD and/or celiac subjects just before a gluten containing meal to be ingested. By means of over-expression of inventive sequences within wheat genome a wheat not toxic for celiac subjects could, moreover, be obtained, said wheat yet maintaining rheological and pasta making properties. In fact, the lack of rheological properties necessary for bread making and pasta making are the main drawbacks of currently available on the market of gluten-free foods. Foods made of flour of maize and rice, although well tolerated by celiac subjects, are not palatable as corresponding wheat made ones. Therefore, said wheat would offer an important innovation for celiac subjects.

It is therefore a specific object of the present invention a peptide consisting of the following sequence:
QQPQ[X]ₙQPF (SEQ ID No: 4)
wherein X is any amino acid;
n is an integer equal or greater than 1, wherein [X]ₙ is RPQ, W, GGG, YYY, GG, or preferably RPQ.

It is a further object of the present invention a nucleotide sequence encoding for the peptide as above defined or a vector comprising said nucleotide sequence. Moreover the invention concerns microorganisms or probiotics comprising as above defined vector. For example said microorganisms can be bacteria or yeasts used for the preparation of bakery products.

The use of probiotics or microorganisms as above defined for the preparation of food for celiac subjects is a further object of the present invention.

The invention concerns, moreover, a pharmaceutical composition comprising or consisting of at least one peptide as above defined or a nucleotide sequence or a vector as above defined, as active principle, in combination with one or more pharmaceutical acceptable excipients and/or adjuvants.

Moreover, the invention concerns a peptide or a pharmaceutical composition as above defined for use in treatment or prevention of celiac disease by inhibiting the toxicity of peptide from amino acid 31 to 43 of gliadin in celiac patients or subjects at high risk to develop celiac disease. Said peptide and pharmaceutical composition can be administered, for example, by oral or intranasal route. In particular said peptide and pharmaceutical composition can be administered before meals.

It is a further aspect of the present disclosure which is not part of the claimed invention, the use of variety of plants like, for example, wheat, barley, rye, oat, naturally expressing peptide SEQ ID NO: 2 (peptide pRPQ) for the preparation of foods for celiac subjects. Plant variety naturally expressing SEQ ID NO: 2 are, for example, *Secale cereale* and tender wheat *Kavzak.*

Among wheat varieties, for example those carrying 1BL/1RS translocation from rye and thus naturally expressing pRPQ peptide, like for example, *Triticum aestivum 1R*/*1S, Barra, Carisma, Colledoro, Collerosso, Dorico, Geppetto, Geronimo, Marvao, Sibilla, Sirmione,* can be used.

The present invention concerns, in addition, transgenic plants, as for example wheat, barley, rye, oat, expressing a peptide as above defined and use thereof for the preparation of food for celiac patients.

The present invention now will be described by an illustrative, but not limitative way, according to preferred embodiments thereof, with detailed reference to enclosed drawings.
**Figure 1****.**
   **Alignment of 5 database found sequences:** (http://appliedbioinformatics.wur.nl/glutendb/tree.htm) as a result of search for sequences containing QQPQDAVQPF (SEQ ID NO: 1) peptide described by Silano et al. (7). Said search detected three ω-secalins, two isolated from *Secale cereale* (X60924 and X60295 accession numbers) and one from *Triticum aestivum* (AF000227) and two ω-gliadins isolated from *Aegilops tauschii* (AAT74547) and *Triticum aestivum* (AAG17702) containing a decapeptide (gray overshadowed) with structural similarities to query sequence. Asterisks indicate sequence identity among said five protein sequences.
**Figure 2****. Phylogenetic tree** constructed with representative sequence of gliadin (prolamin) protein families. Used sequences are indicated by accession number. Within brackets tree spacings are indicated.
**Figure 3****. Multiple alignment sequence** using ClustalW2 of 7 ω-secalin proteins containing "QQPQRPQQPF" (SEQ ID NO: 2) motif (gray underlined). γ1-Sec and Glia-γ2 epitopes (Vader et al., 2003) are indicated in block italics and underlined, respectively. Asterisks indicate sequence identity among seven protein sequences.
**Figure 4****. PCR label specific for alleles containing RPQ motif.** RPQ F/R primer pair produced a fragment of 200 bp for RPQ gene. Lane 1 (*Secale cereale cv. Askari*) and lane 3 (*Triticum aestivum 1R*/*1S cv. Kavkaz*) and no RPQ fragment for 2 (*Triticum durum cv. Adamello*) and 4 (*Triticum aestivum cv. Balance*) lanes.

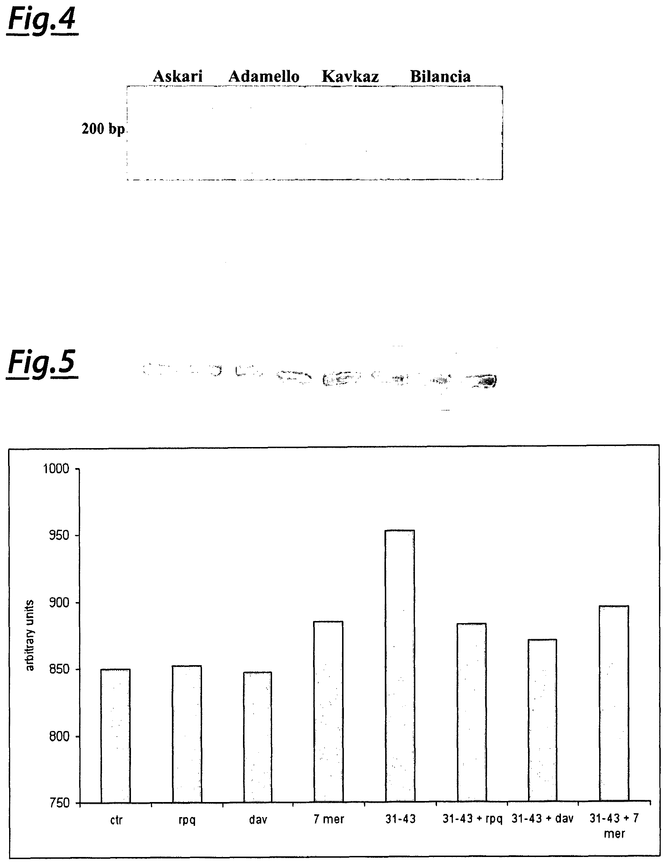

**Figure 5****. Western blotting for expression of ICAM-1 in T84 cells.** Incubation with p31-43 resulted in increase of ICAM-1 level in T84 cells compared to cells incubated with medium only. No meaningful increase of ICAM-1 expression in T84 cells is noticed when said cells are exposed separately to pDAV and pRPQ. Incubation with 7mer resulted in poor increase of ICAM-1 level in cells. Simultaneous exposure of T84 cells to p31-43 toxic peptide and pDAV and pRPQ, respectively, resulted in a ICAM-1 intracellular expression level similar to cells exposed only to culture medium. On the contrary, no meaningful decrease of ICAM-1 expression is detected when cells are incubated with 7mer and p31-43 at the same time, compared to cells exposed only to p31-43. Panel A is representative of a blot. Results are expressed as average ± SEM. The experiments have been carried out in triplicate thrice. The results are compared with ANOVA. (Panel B).
**Figure 6****. Expression levels of ICAM-1 and DR-3 within mucosa of small intestine** incubated with only medium, only gliadin PT digest and pRPQ. RPQ peptide meaningfully prevents gliadin-dependent increase of expression of ICAM-1 and HLA-DR inflammatory molecules.
**Figure 7****. Expression levels of Py99 and p42-44 within mucosa of small intestine** incubated with only medium, only p31-43 and at the same time pRPQ. RPQ peptide meaningfully prevents gliadin-dependent expression increase of Py99 and p42-44 inflammatory molecules.
**Figure 8****. TG2 activity within duodenal mucosa from celiac patients.** RPQ peptide meaningfully prevents the increase of p31-43-induced TG2 activity.

### EXAMPLE 1: Study of protecting activity of pRPQ peptide towards the toxicity of a-gliadin 31-43 peptide and function of spacing between QQPQ and QPF domains of 7mer peptide (SEQ ID NO: 3)

### MATERIALS AND METHODS

### Wheats and RNA isolation

Seeds of Kavkaz tender wheat cultivar (*Triticum aestivum L.,* genome AABBDD) carrying 1BL/1RS translocation from rye (*Secale cereale L.*) are processed in the present study. Samples from seed endosperma are collected 20 days after anthesis and stored at -80°C. Total RNA has been extracted using Trizol reagent (Invitrogen), according to producer instructions. In order to avoid contamination with starch, ground endospermas are treated using a medium consisting of 50 mM Tris, pH 9,00, 200mM NaCl, 1% Sarcosyl, 20 mM EDTA, DTT 5mM and successive extraction using phenol-chloroform-isoamyl alcohol (25:24:1). Purified samples then are analyzed by spectrophotometry, 1 mg of total RNA has been reverse transcribed using 200U of SuperScript II RNAse H reverse transcriptase (Invitrogen) and poly(T) primer according to producer instructions (final volume 20µl).

### PCR

Known DNA sequences (X60294, X60295 and F000227 accession numbers) of ω-secalin genes are used in order oligonucleotide primer to be designed. Forward primer :5'CACAAATCCAACATGAAGACCTTCC3' (SEQ ID NO:5) and reverse primer: 5'CCCGATGCCTATACCACTACTACAA3' (SEQ ID NO:6) have been used to amplify DNA. Forward and reverse primers have been defined as sequences comprising from 12 nucleotides upstream to 8 nucleotides downstream of CDS start and stop codons, respectively.

PCR has been carried out in 25µl final volume samples, containing 1µg total RNA, 1X PCR buffer (Promega) with 1.5 mM MgCl₂, 200 nM each primer, 200 mM dNTPs and 1U GoTaqTM DNA polimerase (Promega). Thermal cycling consisted of a starting denaturation step at 94°C for 6 minutes, followed by 30 cycles at 94°C for 1 min, 60°C for 30 s, 72°C for 2 min. A final step is applied at 72°C for 7 minutes.

Gene-specific primer have been specifically designed in order to amplify ω-secalin gene encoding for RPQ containing sequence:
RPQ/F 5'CCCTCACACCAACCATTTCCCAC3' (SEQ ID NO:7)
RPQ/R 5'CTACTACAAATGGTTGCTGGGGC3' (SEQ ID NO:8).
Forward primer has been designed on the region encoding for RPQ tripeptide. Amplification of these primers has been carried out under the same conditions as for above reported PCR with the exception that DNA concentration was 50 ng.

### GATEWAY cloning system and sequencing analysis

GATEWAY TM system has been used for directional cloning of PCR products within pDonor221 vector by BP reaction (Invitrogen). Interest DNA fragment has been amplified according to two step PCR approach. The first step has been carried out using above described primer modified with the 5' addition of attB tag:
forward 5'-aaaaagcaggctCACAAATCCAACATGAAGACCTTCC-3' (SEQ ID NO:9);
reverse agaaagctgggtCCCGATGCCTATACCACTACTACAA-5'-3' (SEQ ID NO:10)

Second PCR amplification has been carried out using attB primer adapter (Invitrogen) recognizing attB tag:
5'-ggggacaagtttgtacaaaaaagcaggct-3' (SEQ ID NO:11)
5'-ggggaccactttgtacaagaaagctgggt-3' (SEQ ID NO:12).

PCR final products then have been cloned in pDonor221 vector by BP reaction using BP clonase enzymes. The reaction has been blocked by proteinase K and said products mixed with DH5a competent cells (Invitrogen) for transformation. The procedure has been carried out according to producer protocol. All the cells have been placed on LB plate containing 50 mg/ml of kanamycin.

Cloned genes have been sequenced for both strands using ABI Prism TM Big Dye Terminator Cycle Sequencing Kit with ABI Prism 3130 Analyzer TM (PE Applied Biosystems) automated sequencer by means of M13 forward and M13 reverse primer pair. Sequences have been analyzed and translated in amino acid sequences using Vector NTI Explorer vector (version 10). Signal peptides prediction has been carried out using SignalP 3,0 online accessible program (http://www.cbs.dtu.dk/services/SignalP/). Sequences reported in this study are in NCBI-GenBank database with FJ816032-FJ816047 and FJ823438-FJ823444 accession numbers.

### Peptides

The following peptides of p31-43 gliadin have been synthesized by Primm (Milan, Italy) and purity thereof determined by reverse phase HPLC:
pDAV: QQPQDAVQPF (SEQ ID NO: 1);
pRPQ: QQPQRPQQPF (SEQ ID NO: 2);
p7mer: QQPQQPF (SEQ ID NO: 3);
pGGG: QQPQGGGQPF (SEQ ID NO: 13);
pYYY: QQPQYYYQPF (SEQ ID NO: 14);
pW: QQPQWQPF (SEQ ID NO: 15).
hTPO (535-551) LDPLIRGILLARPAKL QV (SEQ ID NO: 16),
control irrelevant peptide.

### T84 and K562(S) cell lines

T84 human colonic carcinoma cells have been cultured under 5% CO₂ atmosphere at 37°C in 50% DMEM, (Gibco, supplied by Invitrogen, Carlsbad, CA, USA) and 50% G12 containing 4,5 g/L glucose, 2 mM L-glutamine, 50 U/ml penicillin, 50 mg/ml streptomycin, 1% essential amino acids, 1% HEPES and 10% bovine fetal serum (FBS).

K562(S) cells, an immature subclone of a cell line from a blastic phase chronic myelogenous leukemia patient, are cultured under 5% CO₂ atmosphere at 37°C in RPMI (Gibco, supplied by Invitrogen, Carlsbad, CA, USA) containing 4,5 g/L glucose, 2 mM L-glutamine, 50 U/ml penicillin, 50 mg/ml streptomycin, 1% essential amino acids, 1% HEPES and 10% bovine fetal serum (FBS).

### T84 cell Culture

T84 cells have been treated with p31-43 peptide in absence and presence of pRPQ, pDAV, p7mer (at peptide concentration of 20 µg/ml) and culture medium only (like negative control) for 24 hours at 37°C.

### K562(S) cell culture and agglutination test

K562(S) cells to be used for agglutination test have been collected by centrifugation and washed two times with calcium and magnesium free PBS. Then cells have been resuspended at concentration of 10⁸ cells/ml in PBS; 25 ml of cell suspension added to each well of a 96 well plate. The peptide has been added at a concentration of 10 µg/ml. The final total volume has been 100 µl. Cell suspension has been incubated at room temp. for 30 minutes. Agglutination rate has been measured using a 96 well plate reader equipped with stirrer. Turbidity of cellular suspension has been measured at 600 nm (OD600 nm) under continuous stirring at 0 and 30 min times. The difference of T0 and T30 readings has been taken as agglutination rate (CV).

### Western blotting

For WB analysis, T84 cells have been seeded at a concentration of 1 x 10⁵ cells/ml in 12 well plate and shortly treated before confluence was reached. Treatment has been carried out using 20 µg/ml of medium for each peptide (p31-43, p7mer, pDAV, pRPQ) for 24 hours. In order the protecting activity of pDAV and pRPQ towards toxic effect of p31-43 to be tested, a pre-incubation for 3 hours using these peptides before the exposure to p31-43, has been performed. After incubation, cells have been treated with buffer containing 150 mM NaCl, 0,05% sodium dodecylsulfate (SDS) and 1% Triton X-100. This mixture then has been centrifuged for 5 minutes at 4°C. Protein concentration has been measured according to Laemmli method. SDS-(Polyacrylamide Gel Electrophoresis) has been carried out using 4% and 7.5% gel. 30 mg of protein have been loaded for each gel run. Then the proteins have been transferred on nitrocellulose membrane (Biorad). Membranes have been incubated overnight with mice anti-ICAM-1 monoclonal Abs (Abcam, Cambridge, UK) at 1:250 dilution. Successively, after washings, said membrane have been incubated with secondary antibody (peroxidase HRP:3000; Biorad). Membrane occurring proteins are detected using chemiluminescent detection kit (Biorad), according to producer instructions. Blot intensity of protein bands has been evaluated by Biorad ChemiDoc densitometer.

### Organ culture

Duodenal mucosa samples have been treated *in vitro* for 3 and 24 hours using medium, gliadin PT digest (500 µg/mL), p31-43 (20 µg/ml), pRPQ (10 µg/ml). P7mer and pTPO (10 µg/ml) have been used in all the experiments as control peptides.

### Immunolocalization

4 µm thick sections of frozen biopsy samples from celiac patients have been fixed in acetone for 10 minutes. Then sections have been incubated for 2 hours at room temp. with following antibodies: anti-phospho-tyrosine PY99 mAb (1:80, IgG2b; Saint Cruz Biotechnology, Saint Cruz, CA), phospho-p42/p44 (1:500, polyclonal Ig; Cell Technology Beverly, USA), ICAM1 (1:500, Abcam, Cambridge, BUT, USA), HLA-DR (1:500, Abcam, Cambridge, BUT, USA). Antigene expression and distribution thereof within biopsy sample have been visualized by indirect immunofluorescence as previously described. Monoclonal isotype control (IgG1 and IgG2) has been used as control. Samples have been analyzed using Zeiss LSM510 confocal laser scanning unit (Carl Zeiss, Germany).

### In situ determination of TG2 activity

7 µm diameter sections have been collected on slide, pre-incubated with buffer (965 ml of 100 mmol/L Tris-HCI, pH 7,4, 25 ml of 200 mmol/L CaCl₂) for 15 minutes, and then pre-incubated with same buffer added with 10 ml of 10 mmol/L of biotinylated mono-dansylcadaverine (bio-MDC) (Molecular Probes, Leiden, The Netherlands) for 1 hour at room temp. The reaction is stopped using 25 mmol/L of EDTA for 5 minutes, then sections have been fixed in 4% paraformaldeide for 10 minutes. Labelled substrate incorporation has been visualized by incubation with streptavidin-conjugated RPE (1:50; Dako) for 30 minutes. Sections have been visualized fluorescently using Axioxop Zeiss 2 Plus micropurpose (Zeiss, Jena, Germany).

### RESULTS

### pRPQ identification

### Database analysis

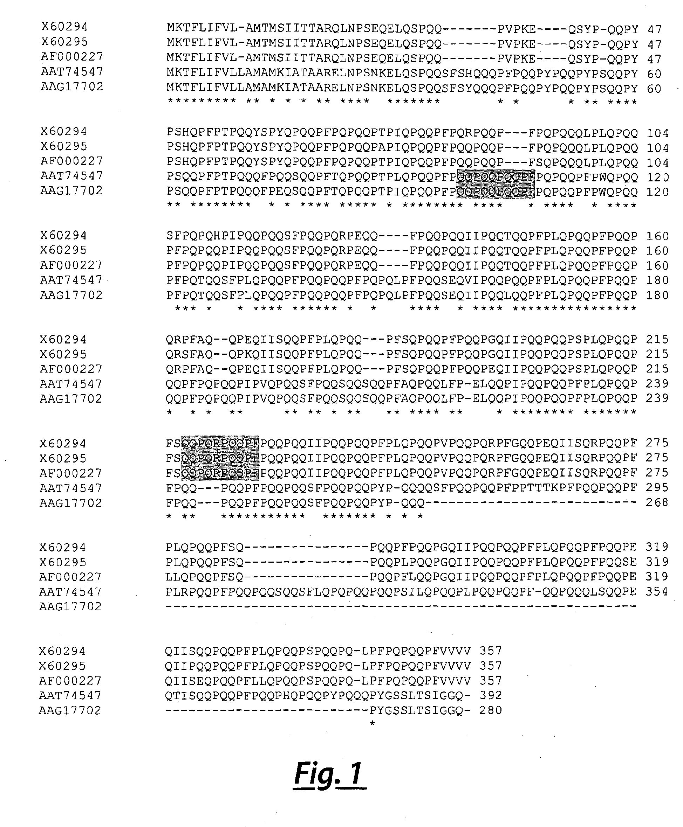

A previous study (7) identified a decapeptide in alcohol-soluble fraction of hard wheat variety (Adamello), said decapeptide being suitable to prevent the activation of peripheral lymphocytes of celiac subjects by gliadin peptides. Based on said knowledge, a bioinformatic search has been carried out in order gene encoding for this sequence to be identified. The search on gluten database (http://appliedbioinformatics.wur.nl/glutendb/tree.html) was unsuccessful because no ORF encoding for a protein sequence containing title peptide was detected. Database analysis in addition identified five sequences with ORFs partially overlapping on pDAV sequence (Figure 1). AAT74547 and AAG17702 accession numbers are referred to coding of ω-gliadin family proteins in *Aegilops tauschii* (12) and *Triticum aestivum* (13). Both the sequences contain a copy of QQPQQPQQPF (SEQ ID NO: 17). Three additional sequences have been identified for the presence of a single copy of QQPQRPQQPF decamer (SEQ ID NO: 2). X60294 and X60295 code for ω-secalin isolated from Secale *cereale* (14), while AF000227 for for ω-secalin isolated from a tender wheat genotype carrying wheat-rye 1BL.1RS translocation (15).

Some structural homologies are detected by comparison of pDAV decapeptide and QQPQRPQQPF sequence (SEQ ID NO: 2). R (arginine) and D (aspartic acid) are both polar amino acids, although with opposite charges (negative for A, positive for R) and both are suitable to form hydrogen bond. P (proline), Q (glutamine), A (alanine) and V (valine) are all neutral amino acids. Based on hypothesis that these structural homologies were sufficient to confer a protecting activity towards CD also to pRPQ peptide, further studies have been carried out in order to characterize pRPQ gene and verify the activity of this peptide for CD.

### Molecular characterization of ω-secalin gene

Based on results from database search, PCR primers have been designed on ω-secalin highly conserved regions found in database and used in order to clone all the ω-secalins expressed in mature endosperma of Kavkaz tender wheat carrying wheat-rye 1BL-1RS translocation. Amplified cDNA have been cloned and more than 100 clones randomly selected from specific ω-secalin library have been sequenced. Within these sequences 23 not redundant genes have been identified.

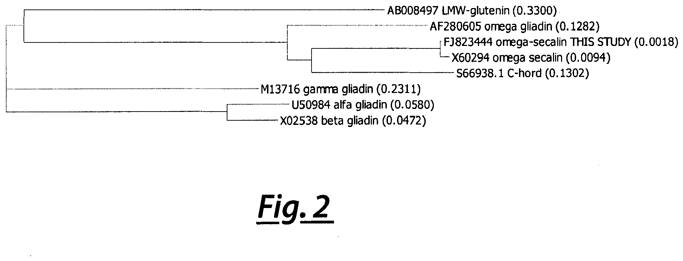

The ORFs of cloned ω-secalin sequences varied from 115 to 357 amino acid residues, the longest corresponding to an already database available sequence with the exception of few amino acid substitutions. This length variability of ω-secalin sequence is according to previously reported studies (16). Primary sequences of ω-secalins cloned in the present study confirm the presence of glutamine and proline rich repeat motifs: PQQPFPQQ (SEQ ID NO: 18) and QQPQQPF (SEQ ID NO: 3), which are very common also in ω-gliadins and C-ordeins. Figure 2 shows philogenetic trees constructed after the alignment of gliadin amino acid sequences, confirming stricter evolutionary relationship of ω-secalin with ω-prolamin, ω-gliadin and C-ordein.

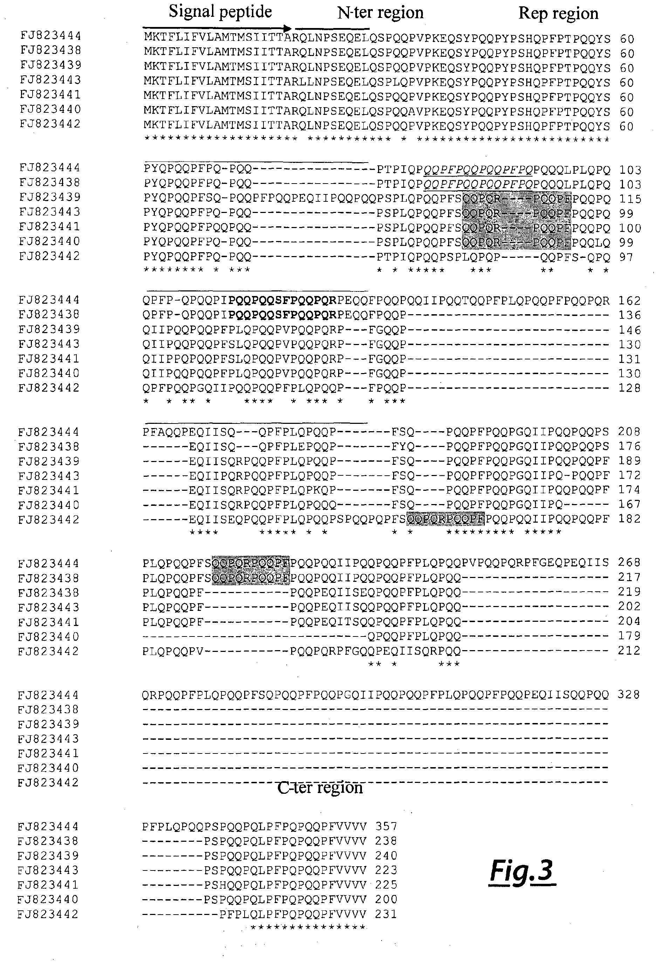

Out of 23 ω-secalins identified in this study, 7 proteins contain QQPQRPQQPF sequence (SEQ ID NO: 2). ORF of these sequences varied between 200 and 357 amino acids and shared high number of sequences with ω-secalins in NCBI database. After the comparison of these 7 sequences with ω-secalins, four main structural regions have been identified: **(a)** a conserved peptide signal of 19 amino acids, **(b)** a N-terminal region of 27 amino acids, **(c)** a repetitive glutamine and proline rich region and **(d)** a C-terminal region of 12 amino acids ending with four valine residues (Figure 3). Signal sequence and C-terminal and N-terminal regions are conserved in all cloned ω-secalins with small differences due to single amino acid substitutions. N-terminal sequences starting with RQL, one of known and already described variants in ω-secalins, ω-gliadins and C-ordeins (17).

Based on scientific literature (11), two antigenic peptides, i.e. Glia-γ2 QQPFPQQPQQPFPQ (SEQ ID Not: 19) and Sec-y1 PQQPQQSFPQQPQR (SEQ ID Not: 20) (aligning with Glia-γ1 contained epitopes), are identified inside of sequence of ω-secalins genes we have cloned. First epitope (Glia-γ2) occurs in 8 ω-secalins, while the second (Sec-y1) occurs in 5 sequences. Moreover, two genes of RPQ (FJ823444 and FJ8234438) triplet containing ω-secalins contain also peptides aligned with two gluten epitopes (Glia-γ2 and γ1-Sec).

### Development of label for identification of RPQ sequence carrying genotypes

(RPQ/F) (RPQ/F 5'CCCTCACACCAACCATTTCCCAC3'-SEQ ID NO:7)) forward primer constructed based on region containing RPQ motif in sequence FJ823444 occurring in ω-secalins has been paired to reverse primer (RPQ/R) (5'CTACTACAAATGGTTGCTGGGGC3'-SEQ NO:8) and used in order to selectively amplify alleles containing RQP motif. RPQ genotype produced a fragment of 200 bp. This dominant label available for selection at Sec-1 locus allows the discrimination of RPQ containing and not containing alleles to be carried out.

### Agglutination of K562(S) cells

Agglutination test on K562(S) cells has been used in order the protecting activity towards p31-43 of pDAV peptides and pRPQ and peptides obtained by addition a spacing in QQ residues at position 4 and 5 of p7mer QQPQQPF (SEQ ID NO: 3), to be tested and screened. As shown in table 1 and expected, p7mer not only does not display any protecting effect towards p31-43, but in addition displays also agglutinant activity towards K562 cells.

Both pDAV and RPQ prevent p31-43 induced agglutination, pDAV being more active than pRPQ. Table 1 shows agglutinating and protecting activity of tested peptides. The results are expressed as SEM ± S.D of difference (Δ) of O.D at 600 nm at 0 and 30 minutes x 100 after incubation of K562(S) cells. * p< 0.05. Statistical analysis has been carried out using Wilcoxon test.

**Table 1**

| | **(Δ) O.D.** |
|---|---|
| **CTRL-medium** | -10 ± 0.9 |
| **p31-43** | 1,8 ± 0.3 |
| **p7mer** | 1,0 ± 0.3 |
| **p31-43 + pDAV** | -12±1.9 |
| **p31-43 + pRPQ** | -9,8 ± 1.7 |
| **31-43 + pRPQ+ pDAV** | -9,5 ± 2.1 |
| **p31-43 + p7mer** | 1,5 ± 1.1 |
| **P31-43 + pGGG** | -7,9 ± 1.4 |
| **P31-43 + pW** | -9,1 ± 2.3 |
| **P31-43 + pGG** | -5,2 ± 0.5 |
| **P31-43** + **pYYY** | -7 ± 2.5 |

### Peptides with 3 aa spacing (QQPQ [X]ₙ QPF wherein n=3)

Two peptides obtained with the insertion of 3 residues within amino acid sequence of p7mer have displayed a protecting activity towards p31-43 induced agglutination of K562 cells, YYY peptide displays little higher protecting activity than pGGG.

### Peptides with 2 aa spacing (QQPQ [X]ₙ QPF wherein n=2)

pGG peptide prevents meaningfully p31-43 induced agglutination of K562 cells, although this activity is lower than peptides with 3 amino acid spacing.

### Peptides with 1 aa spacing (QQPQ [X]ₙ QPF wherein n=1)

Finally peptide obtained inserting one W residue within sequence of p7mer as spacing displays a protecting activity higher than some peptides obtained by insertion of 2 or 3 amino acids within QQ.

### RPQ and DAV peptides down-regulate p31-43-dependent ICAM-1 increase in intestinal epithelial T84 cells

Protecting activity of some peptides selected in an *in vitro* model of intestinal epithelium, i.e. T84 cells, has been confirmed. These cells belong to human adenocarcinoma cell line displaying histological and enzymatic characteristics of human enterocytes. This represents a common and validated model of celiac inflammation. Epithelial inflammation is a fundamental step within cascade of events resulting in immune activation in duodenal mucosa of celiac subjects, therefore the prevention of this event could be a therapeutic strategy for CD, as an alternative to gluten free diet. In the context of this response, a key event is ICAM-1 increased expression.

As shown in figure 5, ICAM-1 is a pro-inflammatory molecule, also involved in some pathways of intracellular signaling and cellular kinase regulation. Exposure to only p31-43 up-regulates the expression of this inflammatory molecule in T84 cells, while simultaneous incubation of these cells with p31-43 and pRPQ or pDAV, respectively, results in ICAM-1 levels similar to cells exposed only to culture medium. Accordingly, p7mer does not exert any anti-inflammatory effect, on the contrary, increases ICAM-1 intracellular levels.

### RPQ peptide controls p31-43-induced mucosal response from in duodenum of celiac subjects

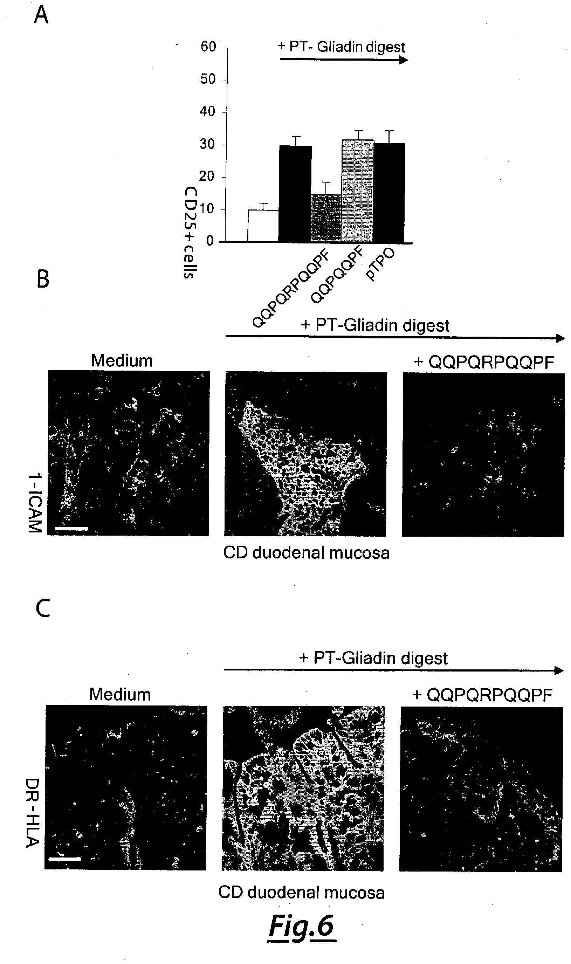

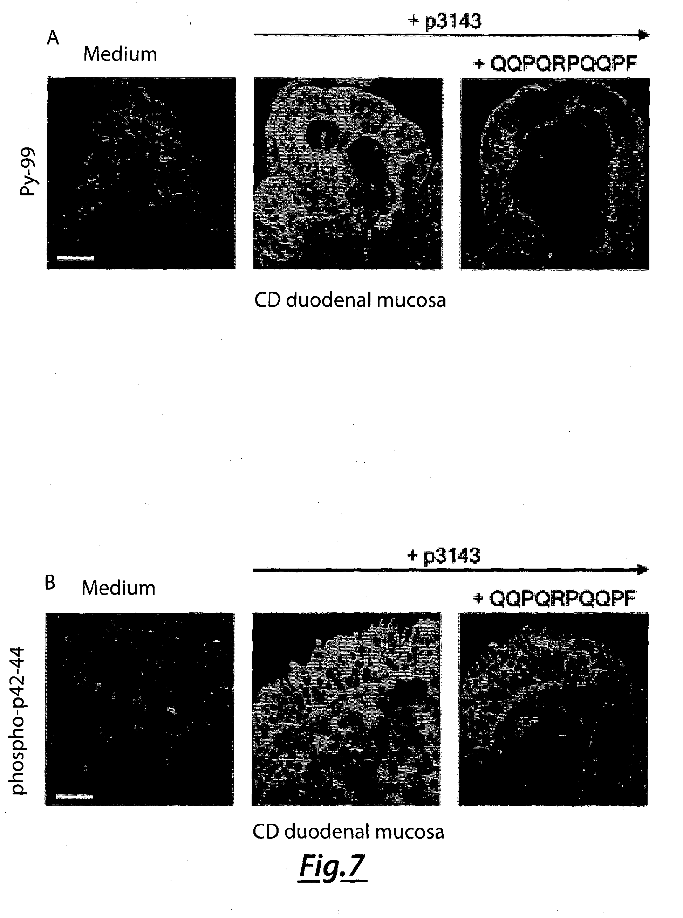

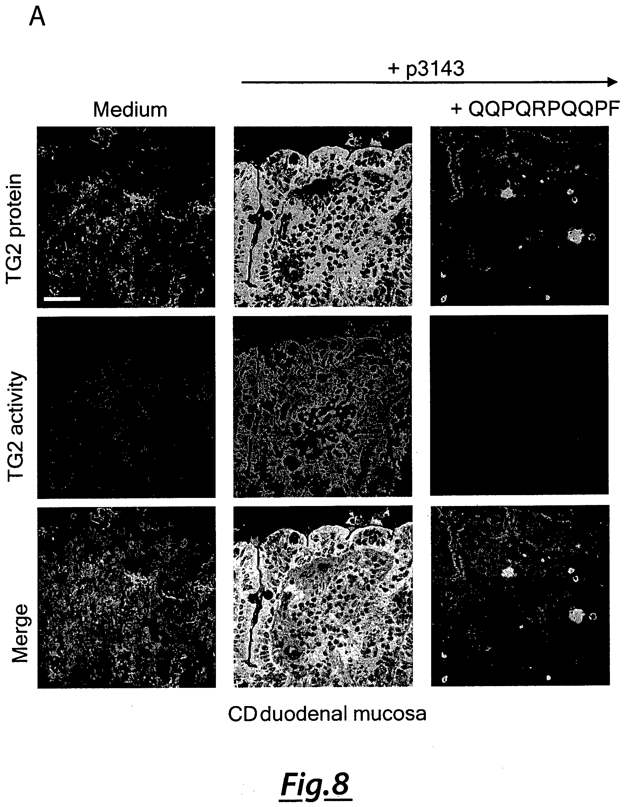

Previously it has been demonstrated that pDAV is suitable to reduce the activation of T cells in celiac patients (8). In order to further explain the biological effect of pDAV and pRPQ and mechanism thereof, it has been investigated whether pRPQ was also effective in controlling immune response induced by gliadin peptides within intestine of celiac subjects. Therefore, intestinal biopsies from celiac patients have been incubated with 500 µg/ml of gliadin PT digest in the presence or absence of 10 µg/ml of pRPQ or p7mer or pTPO (536-547) like irrelevant control peptide. pRPQ, but not control peptides, has been suitable to control gliadin induced increase of T CD25+ cells within lamina propria (Figure 6A), as well as ICAM1 (Figure 6B) and HLA-DR (Figure 6C) expression, i.e. labels of mucosa immune activation (18).
Moreover, in order to evaluate whether pRPQ displays inhibiting activity towards previously described early native immune response (4, 5), biopsies from celiac subjects have been incubated with p31-43 (20 µg/ml) with or without pRPQ peptide (10 µg/ml). Figure 7 shows that pRPQ prevents the increase of native response parameters, like p42/44 phosphorylation induced by p31-43 peptide (Figure 7A and B). These results indicate that pRPQ is suitable to interfere with gliadin exposition induced premature native response. The increase of TG2 activity and function is a characteristic of celiac inflammation (4) promoting the activation of adaptive immune response and a key characteristic of mucosa early activation at gliadin exposure. Therefore the ability of pRPQ to modulate TG2 activity in intestinal biopsies from celiac subjects has been tested. The incubation with p31-43 induces a dramatic up-regulation of TG2 activity in biopsies from celiac subjects while said up-regulation is completely inhibited after co-incubation with pRPQ (Figure 8). TPO control peptide displayed to be lacking of any protecting effect against the activation induced by gliadin or p31-43 while pRPQ is lacking in mucosa immunity stimulating activity in duodenal biopsies from both celiac and healthy control subjects (data not shown).

### BIBLIOGRAPHY

1. Koning F, Schuppan D, Cerf-Bensussan N, Sollid LM. 2005. Pathomechanisms in celiac disease. Best Pract. Res. Clin. Gastroenterol. 2005; 19: 373-87.
2. Green PH, Jabri B. Coeliac disease. Lancet. 2003; 362: 383-91.
3. Jabri, B, Kasarda DD, Green PH. Innate and adaptive immunity: the yin and yang of celiac disease. Immunol. Rev. 2005; 206: 219-31.
4. Maiuri L, Ciacci C, Ricciardelli I, Vacca L, Raia V, Auricchio S, Picard J, Osman M, Quaratino S, Londei M. Association between innate response to gliadin and activation of pathogenic T cells in coeliac disease. Lancet. 200; 362:30-7.
5. Luciani A, Villella VR, Vasaturo A, Giardino I, Pettoello-Mantovani M, Guido S, Cexus ON, Peake N, Londei M, Quaratino S, Maiuri L. Lysosomal accumulation of gliadin p31-43 peptide induces oxidative stress and tissue transglutaminase-mediated PPARgamma downregulation in intestinal epithelial cells and coeliac mucosa. Gut. 2010; 59:311-9
6. De Vincenzi M, Gasbarrini G, Silano V. A small peptide from durum wheat gliadin prevents cell agglutination induced by prolamin-peptides toxic in coeliac disease. Toxicology. 1997; 120:207-13.
7. Silano M, Di Benedetto R, Trecca A, Arrabito G, Leonardi F, De Vincenzi M. A decapeptide from durum wheat prevents celiac peripheral blood lymphocytes from activation by gliadin peptides. Pediatr Res. 2007; 61 :67-71.
8. Silano M, Di Benedetto R, Maialetti F, De Vincenzi A, Calcaterra R, Trecca A, De Vincenzi M. A 10-residue peptide from durum wheat promotes a shift from a Th1-type response toward a Th2-type response in celiac disease. Am J Clin Nutr. 2008; 87: 415-23.
9. Silano M, Leonardi F, Trecca A, Mancini E, Di Benedetto R, De Vincenzi M. Prevention by a decapeptide from durum wheat of in vitro gliadin peptide-induced apoptosis in small-bowel mucosa from coeliac patients. Scand J Gastroenterol. 2007; 42: 786-7.
10. De Vincenzi M, Stammati A, Luchetti R, Silano M, Gasbarrini G, Silano V. Structural specificities and significance for coeliac disease of wheat gliadin peptides able to agglutinate or to prevent agglutination of K562(S) cells. Toxicology. 1998; 127: 97-106.
11. Silano M, Vincentini O, De Vincenzi M. Toxic, immunostimulatory and antagonist gluten peptides in celiac disease. Curr Med Chem. 2009;16:1489-98.
12. Hassani, M.E., Shariflou, M.R., Gianibelli, M.C., Sharp, P.J., Characterisation of a ω-gliadin gene in Triticum tauschii. Journal of Cereal Science 2008; 47: 59-67.
13. Hsia, C.C., Anderson, O.D., 2001. Isolation and characterization of wheat ω-gliadin genes. 2001; 103: 37-44.
14. Hull, G.A., Halford, N.G., Kreis, M., Shewry, P.R.. Isolation and characterization of genes encoding rye prolamins containing a highly repetitive sequence motif. Plant Molecular Biology 1991; 17: 1111-5.
15. Clarke, B.C., Mukai, Y., Appels, R. The Sec-1 locus on the short arm of chromosome 1R of rye (Secale cereale). Chromosome 1996; 105 : 269-275.
16. Clarke, B.C., Apples, R.. Sequence variation at the Sec-1 locus of rye, Secale cereale (Poaceae). Plant Systematics and Evolution 1991; 214: 1-14.
17. Kasarda, D.D., Autran, J.C., Lew, E.J.L., Nimmo, C.C., Shewry, P.R.. N-terminal amino acid sequences of ω-gliadins and ω-secalins. Implications for the evolution of prolamin genes. Biochimica et Biophysica Acta 1983; 747: 138-150.
18. Maiuri L, Ciacci C, Auricchio S, Brown V, Quaratino S, Londei M. Interleukin 15 mediates epithelial changes in celiac disease. Gastroenterology. 2000; 119:996-1006.

## Claims

1. Peptide consisting of the following sequence:
QQPQ [X]n QPF (SEQ ID No: 4)
wherein X is any amino acid;
n is an integer equal or greater than 1, wherein [X]ₙ is RPQ, W, GGG, YYY, GG, or preferably RPQ.

2. Nucleotide sequence encoding for peptide as defined in claim 1.

3. Vector comprising nucleotide sequence as defined in claim 2.

4. Microorganisms or probiotics comprising vector as defined in claim 3.

5. Use of microorganisms or probiotics as defined in claim 4 for the preparation of foods for celiac subjects.

6. Pharmaceutical composition comprising or consisting of at least one peptide as defined in claim 1 or nucleotide sequence as defined in claim 2 or vector as defined in claim 3, as active principle, in combination with one or more pharmaceutical acceptable excipients and/or adjuvants.

7. Peptide consisting of the following sequence:
QQPQ [X]ₙ QPF (SEQ ID No: 4)
wherein X is any amino acid;
n is an integer equal or greater than 1, wherein [X]ₙ is RPQ, W, GGG, YYY, GG, or preferably RPQ, or a pharmaceutical composition comprising or consisting of at least one of said peptide or a nucleotide sequence encoding for said peptide or a vector comprising said nucleotide sequence, as active principle, in combination with one or more pharmaceutical acceptable excipients and/or adjuvants, said peptide or pharmaceutical composition being for use in treatment or prevention of celiac disease by inhibiting the toxicity of peptide from amino acid 31 to 43 of gliadin in celiac patients or subjects at high risk to develop celiac disease.

8. Peptide or pharmaceutical composition as defined in claim 7 for use as defined in claim 7, wherein said peptide or said pharmaceutical composition is administered by oral or intranasal route.

9. Peptide or pharmaceutical composition as defined in claim 7 for use as defined in claim 7, wherein said peptide or said pharmaceutical composition is administered before meals.

10. Transgenic plants expressing a peptide as defined in claim 1.

11. Transgenic plants according to claim 10 selected from wheat, barley, rye, oat.

12. Use of transgenic plants as defined in anyone of claims 10-11 for the preparation of food for celiac subjects.

## Patentansprüche

1. Peptid, bestehend aus der folgenden Sequenz:
QQPQ [X]ₙ QPF (SEQ ID-Nr.: 4)
wobei X für eine Aminosäure steht;
n eine ganze Zahl gleich oder größer als 1 ist,
wobei [X]ₙ RPQ, W, GGG, YYY, GG oder vorzugsweise RPQ ist.

2. Nukleotidsequenzcodierung für Peptid nach Anspruch 1.

3. Vektor, umfassend eine Nukleotidsequenz nach Anspruch 2.

4. Mikroorganismen oder Probiotika, umfassend einen Vektor, nach Anspruch 3.

5. Verwendung von Mikroorganismen oder Probiotika nach Anspruch 4 zur Herstellung von Lebensmitteln für Zöliakie-Patienten.

6. Pharmazeutische Zusammensetzung, umfassend oder bestehend aus mindestens einem Peptid nach Anspruch 1 oder einer Nukleotidsequenz nach Anspruch 2 oder einem Vektor nach Anspruch 3 als Wirkprinzip in Kombination mit einem oder mehreren pharmazeutisch verträglichen Hilfsstoffen und/oder Adjuvantien.

7. Peptid, bestehend aus der folgenden Sequenz:
QQPQ [X]ₙ QPF (SEQ ID-Nr.: 4)
wobei X für eine Aminosäure steht;
n eine ganze Zahl gleich oder größer als 1 ist,
wobei [X]ₙ RPQ, W, GGG, YYY, GG oder vorzugsweise RPQ ist,
oder eine pharmazeutische Zusammensetzung, umfassend oder bestehend aus mindestens einem von Peptid oder eine Nukleotidsequenzcodierung für das Peptid oder einem Vektor, umfassend die Nukleotidsequenz als Wirkprinzip, in Kombination mit einem oder mehreren pharmazeutisch verträglichen Hilfsstoffen und/oder Adjuvantien, wobei das Peptid oder die pharmazeutische Zusammensetzung zur Verwendung bei der Behandlung oder Vorbeugung von Zöliakie durch Hemmung der Toxizität des Peptids von Aminosäure 31 bis 43 von Gliadin bei Zöliakie-Patienten oder Personen mit hohem Risiko, Zöliakie zu entwickeln ist.

8. Peptid oder pharmazeutische Zusammensetzung nach Anspruch 7 zur Verwendung nach Anspruch 7, wobei das Peptid oder die pharmazeutische Zusammensetzung auf oralem oder intranasalem Weg verabreicht wird.

9. Peptid oder pharmazeutische Zusammensetzung nach Anspruch 7 zur Verwendung nach Anspruch 7, wobei das Peptid oder die pharmazeutische Zusammensetzung vor den Mahlzeiten verabreicht wird.

10. Transgene Pflanzen zur Expression eines Peptids nach Anspruch 1.

11. Transgene Pflanzen nach Anspruch 10, ausgewählt aus Weizen, Gerste, Roggen, Hafer.

12. Verwendung von transgenen Pflanzen nach einem der Ansprüche 10 bis 11 zur Herstellung von Lebensmitteln für Zöliakie-Patienten.

## Revendications

1. Peptide consistant en la séquence suivante : QQPQ [X]ₙ QPF (SEQ ID N° : 4)
dans lequel X est un quelconque acide aminé ;
n est un entier égal ou supérieur à 1, dans lequel [X]ₙ est RPQ, W, GGG, YYY, GG, ou de préférence RPQ.

2. Codage de séquence de nucléotides pour le peptide tel que défini dans la revendication 1.

3. Vecteur comprenant une séquence de nucléotides telle que définie dans la revendication 2.

4. Micro-organismes ou probiotiques comprenant un vecteur tel que défini dans la revendication 3.

5. Utilisation de micro-organismes ou de probiotiques tels que définis dans la revendication 4 pour la préparation de nourriture pour des sujets cœliaques.

6. Composition pharmaceutique comprenant ou consistant en au moins un peptide tel que définie dans la revendication 1 ou séquence de nucléotides telle que définie dans la revendication 2 ou vecteur tel que défini dans la revendication 3, en tant que principe actif, en combinaison avec un ou plusieurs excipients et/ou adjuvants pharmaceutiques acceptables.

7. Peptide consistant en la séquence suivante :
QQPQ [X]ₙ QPF (SEQ ID N° : 4) dans lequel X est un quelconque acide aminé ;
n est un entier égal ou supérieur à 1, dans lequel [X]ₙ est RPQ, W, GGG, YYY, GG, ou de préférence RPQ,
ou une composition pharmaceutique comprenant ou consistant en au moins un parmi ledit peptide ou une séquence de nucléotides codant pour ledit peptide ou un vecteur comprenant ladite séquence de nucléotides, en tant que principe actif, en combinaison avec un ou plusieurs excipients et/ou adjuvants pharmaceutiques acceptables, ledit peptide ou composition pharmaceutique étant destiné(e) à une utilisation dans le traitement ou la prévention de la maladie cœliaque en inhibant la toxicité du peptide de l'acide aminé 31 à 43 de la gliadine chez des patients cœliaques ou des sujets à haut risque de développer la maladie cœliaque.

8. Peptide ou composition pharmaceutique tel/telle que défini(e) dans la revendication 7 pour une utilisation telle que définie dans la revendication 7, dans laquelle ledit peptide ou ladite composition pharmaceutique est administré(e) par voie orale ou intranasale.

9. Peptide ou composition pharmaceutique tel/telle que défini(e) dans la revendication 7 pour une utilisation telle que définie dans la revendication 7, dans laquelle ledit peptide ou ladite composition pharmaceutique est administré(e) avant les repas.

10. Plantes transgéniques synthétisant un peptide tel que défini dans la revendication 1.

11. Plantes transgéniques selon la revendication 10 sélectionnées parmi le blé, l'orge, le seigle et l'avoine.

12. Utilisation de plantes transgéniques telles que définies selon l'une quelconque des revendications 10 à 11 pour la préparation de nourriture pour des sujets cœliaques.


### SEQUENCE LISTING

<110> Istituto Superiore di sanità
   Consiglio per la Ricerca e la sperimentazione in Agricoltura
<120> Peptides having protective effect towards the inflammatory activity of peptide 31-43 of a-gliadin in celiac disease
<130> PCT28738
<150> RM2011A000487
   <151> 2011-09-19
<160> 20
<170> PatentIn version 3.5
<210> 1
   <211> 10
   <212> PRT
   <213> artificial
<220>
   <223> mutated omega-secalin peptide
<400> 1
   Gln Gln Pro Gln Asp Ala Val Gln Pro Phe
   1               5                   10
<210> 2
   <211> 10
   <212> PRT
   <213> artificial
<220>
   <223> peptide corresponding ro omega-secalin from aa 218 to aa 227 codified by the sequence deposited at NCBI with accession number FJ823438
<400> 2
   Gln Gln Pro Gln Arg Pro Gln Gln Pro Phe
   1               5                   10
<210> 3
   <211> 7
   <212> PRT
   <213> artificial
<220>
   <223> toxic gliadin peptide from aa 139 to aa 145 of omega-gliadin sequence having accession number AAT74547
<400> 3
   Gln Gln Pro Gln Gln Pro Phe
   1               5
<210> 4
   <211> 8
   <212> PRT
   <213> artificial
<220>
   <223> peptide wherein x is one or more of any amino acid
<220>
   <221> misc_feature
   <222> (5)..(5)
   <223> xaa can be any naturally occurring amino acid
<400> 4
   Gln Gln Pro Gln Xaa Gln Pro Phe
   1               5
<210> 5
   <211> 25
   <212> DNA
   <213> artificial
<220>
   <223> forward primer for the amplification of omega-secalin gene
<400> 5
   cacaaatcca acatgaagac cttcc 25
<210> 6
   <211> 25
   <212> DNA
   <213> artificial
<220>
   <223> reverse primer for the amplification of omega-secalin gene
<400> 6
   cccgatgcct ataccactac tacaa 25
<210> 7
   <211> 23
   <212> DNA
   <213> artificial
<220>
   <223> forward primer for amplifying the sequence encoding SEQ ID NO:2
<400> 7
   ccctcacacc aaccatttcc cac 23
<210> 8
   <211> 23
   <212> DNA
   <213> artificial
<220>
   <223> reverse primer for amplifying the sequence encoding SEQ ID NO:2
<400> 8
   ctactacaaa tggttgctgg ggc 23
<210> 9
   <211> 37
   <212> DNA
   <213> artificial
<220>
   <223> forward primer for cloning omega-secalin gene
<400> 9
   aaaaagcagg ctcacaaatc caacatgaag accttcc 37
<210> 10
   <211> 37
   <212> DNA
   <213> artificial
<220>
   <223> reverse primer for cloning omega-secalin gene
<400> 10
   agaaagctgg gtcccgatgc ctataccact actacaa 37
<210> 11
   <211> 29
   <212> DNA
   <213> artificial
<220>
   <223> forward primer for cloning omega-secalin gene
<400> 11
   ggggacaagt ttgtacaaaa aagcaggct 29
<210> 12
   <211> 29
   <212> DNA
   <213> artificial
<220>
   <223> reverse primer for cloning omega-secalin gene
<400> 12
   ggggaccact ttgtacaaga aagctgggt 29
<210> 13
   <211> 10
   <212> PRT
   <213> artificial
<220>
   <223> peptide corresponding to SEQ ID NO:4 sequence wherein X is three G
<400> 13
   Gln Gln Pro Gln Gly Gly Gly Gln Pro Phe
   1               5                   10
<210> 14
   <211> 10
   <212> PRT
   <213> artificial
<220>
   <223> peptide corresponding to SEQ ID No:4 sequence wherein X is three Y
<400> 14
   Gln Gln Pro Gln Tyr Tyr Tyr Gln Pro Phe
   1               5                   10
<210> 15
   <211> 8
   <212> PRT
   <213> artificial
<220>
   <223> peptide corresponding to SEQ ID NO:4 sequence wherein X is W
<400> 15
   Gln Gln Pro Gln Trp Gln Pro Phe
   1               5
<210> 16
   <211> 18
   <212> PRT
   <213> artificial
<220>
   <223> control irrilevant peptide
<400> 16
   Leu Asp Pro Leu Ile Arg Gly Ile Leu Leu Ala Arg Pro Ala Lys Leu
   1 5 10 15
   Gln Val
<210> 17
   <211> 10
   <212> PRT
   <213> artificial
<220>
   <223> peptide having sequence from aminoacid 99 to aminoacid 107 of omega-gliadin with accession number AAT74547
<400> 17
   Gln Gln Pro Gln Gln Pro Gln Gln Pro Phe
   1 5 10
<210> 18
   <211> 8
   <212> PRT
   <213> artificial
<220>
   <223> peptide from aminoacid 74 to aminoacid 81 of omega-secalin having sequence with accession number x60295
<400> 18
   Pro Gln Gln Pro Phe Pro Gln Gln
   1 5
<210> 19
   <211> 14
   <212> PRT
   <213> artificial
<220>
   <223> Glia-gamma2
<400> 19
   Gln Gln Pro Phe Pro Gln Gln Pro Gln Gln Pro Phe Pro Gln
   1 5 10
<210> 20
   <211> 14
   <212> PRT
   <213> artificial
<220>
   <223> sec-gamma1
<400> 20
   Pro Gln Gln Pro Gln Gln Ser Phe Pro Gln Gln Pro Gln Arg
   1               5                   10